# EUROPEAN PATENT SPECIFICATION

(11) **EP 2 080 536 B1**
(45) Date of publication and mention of the grant of the patent: **13.06.2018**
(21) Application number: 08021169.1
(22) Date of filing: 05.12.2008
(51) Int. Cl.: A61K 8/37, A61Q 1/12

(54) **Process for the preparation of cosmetics**
Herstellungsverfahren für Kosmetika
Procédé de préparation de cosmétiques

(30) Priority: 14.12.2007 IT MI20072336
(43) Date of publication of application: 22.07.2009
(73) Proprietor: CHROMAVIS S.p.A., 20122 Milan (IT)
(72) Inventor: Barbieri, Giovanni, 26010 Vaiano Cremasco CR (IT); Carniti, Gian Paolo, 26010 Vaiano Cremasco CR (IT)
(74) Representative: Ripamonti, Enrico

(56) References cited:
- WO-A1-86/00798
- WO-A1-03/055453
- FR-A1- 2 855 404
- FR-A1- 2 883 733

## Description

The present invention concerns a new process for the preparation of cosmetic products. In particular, the invention concerns a process for the preparation of a decorative cosmetic product, for example a make-up product, eye shadow, face powder or blusher having a solid consistency and excellent sheen, with powdery but long lasting characteristics.

### Technical background

Cosmetic products for face and body make-up such as blusher and eye shadow are known. These products normally exist in the form of powders, compact or loose, which have several drawbacks. For example, pressed powders tend to crumble after they have been used for a while, dirtying the container and the items with which they come into contact, while the application of loose powders requires a certain skill and their use is therefore substantially limited to the professional field.

The international application WO03/055453, in the name of the same applicant, describes a process for the preparation of cosmetic products for make-up which comprises extrusion and drying of a mixture obtained by mixing a powdery phase and a fatty emulsion. The cosmetic compounds thus obtained have a good sheen and are so very compact that they can be considered and treated as solids.

### Summary of the invention

It has now surprisingly been found that it is possible to obtain a cosmetic product having characteristics of sheen, compactness and long lasting similar to the extruded compounds as per the international application WO03/055453 via a multi-phase mixing process, but avoiding the extrusion stage.

### Description of the invention

Thus, according to one of its aspects, the present invention concerns a process for the preparation of a cosmetic product which consists in mixing of the following three phases:
- an emulsion of fats for cosmetic use;
- a mixture of colouring powders; and
- a solvent for cosmetic use;

Optionally the mixture thus obtained is sized. The thus obtained is placed over another cosmetic product and dried.

The emulsion of fats for cosmetic use or "fatty emulsion" according to the present invention can be obtained by treating fats for cosmetic use with a neutral or coloured diluent, for example water or any diluent suitable for cosmetic use, including their mixtures; the essential characteristic of the diluent used is that it can be eliminated by drying, advantageously at low temperatures, preferably at room temperature. Water, for obvious reasons of availability and cost, represents a preferred diluent according to the present invention.

According to the present invention, the expression "fats for cosmetic use" designates any fatty material suitable for the preparation of cosmetic products such as the esters of fatty acids, triglycerides, waxes, derivatives and extracts of fruit and seed oils etc. as well as their mixtures.

Useful fats for cosmetic use according to the invention are for example sorbitan stearate, isopropyl stearate, caprylic/capric triglycerides, dipentaerythrityl hexahydroxystearate/stearate rosinate (currently sold under the trademark Cosmol 168AR), magnesium myristate, olive oil and their mixtures.

According to the present invention, the fatty emulsion is prepared by mixing 0.5-3 parts by weight of fats for cosmetic use as defined above with 7-9 parts by weight of diluent, for example 1 part by weight of fats for cosmetic use with 8 parts by weight of diluent, advantageously water.

According to the present invention, the expression "colouring powders" designates any powder, or mixture of powders, containing colouring pigments suitable for cosmetic use.

Suitable colouring powders are for example those obtained by mixing synthetic and/or natural matte or pearly pigments, optionally with inert non-coloured powders, for example mica or talc, in varying quantities according to the powdery effect and colouring power required.

Examples of pearly and colouring substances that can be used include the following:
TiO2 (CI 77891) + mica (CI 77019)
Bismuth oxychloride CI 77163
Mica CI 77019
Copper and bronze powder CI 7740
Iron oxide CI 77491-2-9
Ultramarine blue CI 77007
Manganese violet CI 77742
Hydrated chromium oxide CI 77289
Anhydrous chromium oxide CI 77288
Ferric ferrocyanide CI 77510
Titanium dioxide CI 77891
D&C red no. 7 Ca lake CI 15850:1
D&C red no. 19 Al lake CI 45170:3
D&C red no. 6 Ba lake CI 15850:2
D&C red no. 3 Al lake CI 45430:1 1
D&C red no. 9 Ba lake CI 15585:1
D&C red no. 21 Al lake CI 45380:3
D&C yellow no. 5 Al lake CI 19140:1 1
D&C red no. 30 Al lake CI 73360
D&C yellow no. 10 Al lake CI 47005:1 1
D&C red no. 27 Al lake CI 45410:2
D&C yellow no. 5 Al lake CI 19140:1
D&C orange 5 CI 45370:1 1
FD&C yellow no. 6 Al lake CI 15985:1
FD&C blue no. 1 Al lake CI 42090:2
D&C red 36 CI 12085
Carmine CI 75470.

The colouring powders are generally prepared by mixing the desired pigments with inert materials such as talc, silica and mica.

According to a preferred aspect, preservatives are added to the mixture, for example parabens and sodium dehydroacetate and similar, which have the function of protecting, from a microbiological point of view, the entire product during all the work phases and during storage and use. Said components are advantageously mill-ground for a few minutes to obtain a uniform powder.

Other components can also be added, inert or non-inert materials, to the colouring powder and/or fatty emulsion phases of the invention. By way of example, additives can be added as solid and liquid preserving agents such as sodium benzoate, thickeners such as starch and its derivatives, gelling agents, adhesives in order to make the paste obtained by mixing of the two phases suitable for extrusion, various inert powders, fragrances etc. if required. Said additives, and likewise their properties and uses in the field of cosmetics, are well known to a person skilled in the art.

The expression "solvent for cosmetic use" designates according to the present invention any liquid compatible with cosmetic use which can be easily eliminated during drying. A particularly preferred solvent is water, preferably water purified according to the conventional methods, well known to a person skilled in the art, for example by reverse osmosis, microfiltration, potabilization, etc.

According to a preferred embodiment, in the process of the present invention the above-mentioned phases are mixed respectively in the following amounts:
- 20 to 60%, preferably 30 to 50%, for example approximately 40% of "fatty emulsion";
- 20 to 60%, preferably 30 to 50%, for example approximately 40% of "colouring powders"; and
- 10 to 30%, preferably 15 to 25%, for example approximately 20% of "solvent for cosmetic use"
to give a mixture, said quantities being expressed as percentages by weight with respect to the total weight of the mixture obtained, before drying.

The drying stage of the process of the invention can be performed according to the conventional techniques, for example in an oven, in a vacuum or by fluid bed, but it is preferably performed in the air, at room temperature. Slow drying in the air allows the diluent and solvent to be gradually released from the mixture, thus providing a well-compacted end product with uniform grain.

Advantageously, drying is performed in the air, at room temperature, until almost total evaporation of the diluent and solvent (advantageously consisting, as said, of water), for example until the residual humidity is below or equal to 5%, for example between 1 and 5% (w/w).

According to a particularly preferred embodiment, the invention concerns a process for the preparation of a cosmetic product which comprises:
- preparing a mixture by mixing an emulsion of fats for cosmetic use with colouring powders, advantageously in the relative quantities mentioned above;
- adding to the mixture thus obtained a solvent for cosmetic use, advantageously water, in the respective quantities mentioned above;
- optionally sizing; and
- drying the mixture, advantageously in the air, until obtaining a residual humidity lower than or equal to 5%.

The drying time depends naturally on the temperature and humidity conditions, and the dimensions of the product to be dried. Normally a few hours at room temperature (20-25°C) are sufficient to obtain the desired cosmetic product, with a humidity content below 5%.

Once drying has been completed, the cosmetic product can be visually checked for any defects.

The cosmetic products prepared in this way can be further processed, for example to reduce their dimensions or modify their form. Said further operations can be performed manually or by means of specific machine tools.

The process of the invention thus provides cosmetic products, in particular for make-up, having excellent characteristics of compactness and sheen, at the same time eliminating the stage of extrusion of the mixture.

It is understood that the elimination of an operation that requires specific machinery is a great advantage in terms of economy of the work process.

The process of the invention therefore allows decorative cosmetic products to be obtained, for example for decorative or curative make-up, with the following characteristics:
- optimal consistency, permitting direct application
- an excellent sheen and, at the same time,
- a creamy finish even though the products are powder-based.

The products obtained with the process of the invention therefore represent a valid alternative to the conventional products in powder, loose or compacted, and to the creamy type products.

The following examples aim to illustrate the invention without limiting it in any way. It is understood that technically equivalent alternatives within the reach of a person averagely skilled in the art, even if not explicitly indicated, fall within the scope of protection of the invention. By way of example, the order of preparation and mixing of the ingredients in the process can be arbitrarily modified while obtaining products with the same characteristics.

### Experimental section

### EXAMPLE 1 (out of the claimed scope)

### GENERAL OPERATING METHOD

Clean and appropriately sanitise all the machinery necessary for the work process.

### Preparation of the fatty emulsion

Weigh the water in an emulsifier; add the chosen preservative and mix until fully dispersed. Add any thickeners and the mixture of liquid preservatives and disperse with a homogenizer. Weigh and melt the fatty mass (stearates, triglycerides, etc.) to approximately 60°C, add it to the previously prepared mixture and homogenize. Add gelling agent and work for a few minutes. Check the viscosity and discharge into clean drums.

### Preparation of the colouring powders

Weigh the inert materials, binders and any preservatives (talc, parabens, etc.) and mill-grind for a few minutes. Weigh and add the raw materials that make up the required colour and mill-grind until a uniform mixture is obtained.

### Mixing the mixture

Mix the emulsion of fats, the colouring powders and the solvent for cosmetic use in a mixing machine for approximately 10 minutes. Size in suitable moulds and leave to dry at room temperature.

### EXAMPLE 2

Quali-quantitative composition of a product according to the invention, after drying

| Component | % by weight with respect to final composition |
|---|---|
| Water | 0.80 |
| Myritol© 318 (triglyceride of caprylic/capric acid) | 3.10 |
| Maize starch | 3.10 |
| Dry-flo© pc (aluminium starch octenylsuccinate) | 3.10 |
| Cosmol© 168 ARV (dipentaerythrityl hexahydroxystearate/stearate rosinate) | 2.56 |
| Ceraphyl© 847 (octyldodecyl stearoyl stearate) | 2.48 |
| Isopropyl isosterarate | 2.48 |
| Crill©3 (1,4-anhydro-D-glucitol, 6 octadecanoate) | 1.94 |
| Sepigel© 305 (polyacrylamide/ isoparaffin/lauryl alcohol) | 1.24 |
| Uniphem© P-23 (mixture of preservatives) | 0.62 |
| Sodium benzoate | 0.39 |
| Mixture of colouring powders | 78.19 |

## Claims

1. Process for the preparation of a make-up product which consists in mixing the following three phases:
- an emulsion of fats for cosmetic use;
- a mixture of colouring powders; and
- a solvent for cosmetic use;
optionally sizing the mixture thus obtained,
placing the mixture over another cosmetic product and drying the mixture.

2. Process as claimed in claim 1, wherein said fats are chosen from esters of fatty acids, triglycerides, waxes, fruit and seed oil extracts and their mixtures.

3. Process as claimed in claim 2, wherein said fats are chosen from sorbitan stearate, isopropyl stearate, caprylic/capric triglycerides, dipentaerythrityl hexahydroxystearate/stearate rosinate, magnesium myristate, olive oil and their mixtures.

4. Process as claimed in claims 2 to 3, wherein said emulsion furthermore comprises one or more solid or liquid additives chosen from preserving agents, thickeners, adhesives and gelling agents.

5. Process as claimed in claims 1 to 4, wherein said colouring powders comprise synthetic and/or natural matte or pearly pigments, optionally mixed with inert non-coloured powders.

6. Process as claimed in claims 1 to 5, wherein the drying is performed in the air and at room temperature.

7. Process as claimed in claim 6, wherein the drying is performed until the residual humidity of the cosmetic product is below or equal to 5%.

8. Process as claimed in claims 1 to 7, **characterised in that** 20 to 60% by weight with respect to the total weight of the mixture obtained of "fatty emulsion", 20 to 60% by weight with respect to the total weight of the mixture obtained of "colouring powders" and 10 to 30% by weight with respect to the total weight of the mixture obtained of solvent for cosmetic use are mixed before drying.

9. Process as claimed in claim 8, **characterised in that** 30 to 50% by weight with respect to the total weight of the mixture obtained of "fatty emulsion", 30 to 50% by weight with respect to the total weight of the mixture obtained of "colouring powders" and 15 to 25% by weight with respect to the total weight of the mixture obtained of solvent for cosmetic use are mixed before drying.

10. Process as claimed in claim 9, **characterised in that** approximately 40% by weight with respect to the total weight of the mixture obtained of "fatty emulsion", approximately 40% by weight with respect to the total weight of the mixture obtained of "colouring powders" and approximately 20% by weight with respect to the total weight of the mixture obtained of solvent for cosmetic use are mixed before drying.

## Patentansprüche

1. Herstellungsverfahren für ein Schminkprodukt, bestehend aus dem Mischen der folgenden drei Phasen:
- einer Emulsion von Fetten zur kosmetischen Verwendung;
- einer Mischung von pulverförmigen Farbstoffen; und
- eines Lösungsmittels zur kosmetischen Verwendung;
gegebenenfalls durch die Dimensionierung der so erhaltenen Mischung, die Anordnung der Mischung oberhalb eines anderen kosmetischen Mittels und die Trocknung der Mischung.

2. Verfahren nach Anspruch 1, worin die genannten Esteren aus Fettsäuren, Triglyceriden, Wachsen, Frucht- und Samenölextrakten sowie Mischungen davon ausgewählt werden.

3. Verfahren nach Anspruch 2, worin die genannten Fette aus Sorbitanstearat, Isopropylstearat, Capryl-/Caprin-Triglyceriden, Dipentaerythrityl-hexahydroxystearat/stearat rosinat, Magnesiummyristat, Olivenöl und Mischungen davon ausgewählt werden.

4. Verfahren nach Ansprüchen 2 bis 3, worin die genannte Emulsion weiter eine oder mehrere feste oder flüssige Zusatzstoffe umfasst, die aus Konservierungsmitteln, Verdickungsmitteln, Klebstoffen und Geliermitteln ausgewählt werden.

5. Verfahren nach Ansprüchen 1 bis 4, worin die genannten pulverförmigen Farbstoffe synthetische und/oder natürliche matte oder perlmuttglänzende, gegebenenfalls mit inerten, ungefärbten Pulvern gemischte Pigmente umfassen.

6. Verfahren nach Ansprüchen 1 bis 4, worin die Trocknung im Freien und bei Raumtemperatur durchgeführt wird.

7. Verfahren nach Anspruch 6, worin die Trocknung durchgeführt wird, bis die Restfeuchtigkeit des kosmetischen Mittels weniger als oder gleich 5% ist.

8. Verfahren nach Ansprüchen 1 bis 7, **dadurch gekennzeichnet, dass** 20 bis 60 Gew.-% bezüglich des gesamten Gewichts der erhaltenen Mischung von "Fettemulsion", 20 bis 60 Gew.-% bezüglich des gesamten Gewichts der erhaltenen Mischung von "pulverförmigen Farbstoffen" und 10 bis 30 Ges.-% bezüglich des gesamten Gewichts der erhaltenen Mischung von Lösungsmittels zur kosmetischen Verwendung vor der Trocknung gemischt werden.

9. Verfahren nach Anspruch 8, **dadurch gekennzeichnet, dass** 30 bis 50 Ges.-% bezüglich des gesamten Gewichts der erhaltenen Mischung von "Fettemulsion", 30 bis 50 Gew.-% bezüglich des gesamten Gewichts der erhaltenen Mischung von "pulverförmigen Farbstoffen" und 15 bis 25 Gew.-% bezüglich des gesamten Gewichts der erhaltenen Mischung von Lösungsmittels zur kosmetischen Verwendung vor der Trocknung gemischt werden.

10. Verfahren nach Anspruch 9, **dadurch gekennzeichnet, dass** etwa 40 Ges.-% bezüglich des gesamten Gewichts der erhaltenen Mischung von "Fettemulsion", etwa 40 Gew.-% bezüglich des gesamten Gewichts der erhaltenen Mischung von "pulverförmigen Farbstoffen" und etwa 20 Ges.-% bezüglich des gesamten Gewichts der erhaltenen Mischung von Lösungsmittels zur kosmetischen Verwendung vor der Trocknung gemischt werden.

## Revendications

1. Procédé de préparation d'un produit de maquillage constitué par les trois phases suivantes :
- une émulsion de graisses pour emploi cosmétique ;
- un mélange de poudres colorantes ; et
- un solvant pour emploi cosmétique ;
le cas échéant, dimensionnant le mélange ainsi obtenu, positionnant le mélange au-dessus d'un autre produit cosmétique et séchant le mélange.

2. Procédé selon la revendication 1, où lesdites graisses sont choisies entre acides gras, triglycérides, cires et dérivés, extraits d'huiles de fruits et de graines et leurs mélanges.

3. Procédé selon la revendication 2, où lesdites graisses sont choisies entre stéarate de sorbitan, stéarate d'isopropyle, triglycérides capryliques/capriques, hexahydroxystéarate/stéarate/rosinate de dipentaérythrityle, myristate de magnésium, huile d'olive et leurs mélanges.

4. Procédé selon les revendications 2 ou 3, où ladite émulsion comprend aussi un ou plusieurs additifs solides ou liquides choisis entre agents conservateurs, épaississants, adhésifs et agents gélifiants.

5. Procédé selon les revendications 1 à 4, où lesdites poudres colorantes comprennent des pigments mats ou nacrés synthétiques et/ou naturels, le cas échéant mélangés avec des poudres inertes non colorantes.

6. Procédé selon les revendications 1 à 5, où le séchage est exécuté en l'air et à température ambiante.

7. Procédé selon la revendication 6, où le séchage est réalisé jusqu'à ce que l'humidité résiduelle du produit cosmétique est inférieure ou égale à 5 %.

8. Procédé selon les revendications 1 à 7, **caractérisé en ce que** 20 à 60 % en poids par rapport au poids total du mélange obtenu d' « émulsion grasse », 20 à 60 % en poids par rapport au poids total du mélange obtenu de « poudres colorantes » et 10 à 30 % en poids par rapport au poids total du mélange obtenu de solvant pour emploi cosmétique sont mélangés avant le séchage.

9. Procédé selon la revendication 8, **caractérisé en ce que** 30 à 50 % en poids par rapport au poids total du mélange obtenu d' « émulsion grasse », 30 à 50 % en poids par rapport au poids total du mélange obtenu de « poudres colorantes » et 15 à 25 % en poids par rapport au poids total du mélange obtenu de solvant pour emploi cosmétique sont mélangés avant le séchage.

10. Procédé selon la revendication 9, **caractérisé en ce que** approximativement 40% en poids par rapport au poids total du mélange obtenu d'«émulsion grasse», approximativement 40% en poids par rapport au poids total du mélange obtenu de «poudres colorantes» et approximativement 20% en poids par rapport au poids total du mélange obtenu de solvant pour emploi cosmétique sont mélangés avant le séchage.
